# EUROPÄISCHE PATENTSCHRIFT

(11) **EP 1 989 177 B1**
(45) Veröffentlichungstag und Bekanntmachung des Hinweises auf die Patenterteilung: **01.08.2012**
(21) Anmeldenummer: 07722858.3
(22) Anmeldetag: 20.02.2007
(51) Int. Cl.: C07C 233/03, C07C 231/08

(54) **AMIDOADAMANTANE UND VERFAHREN ZU IHRER HERSTELLUNG**
AMIDOADAMANTANES AND METHOD FOR PRODUCING THE SAME
AMIDOADAMANTANES ET LEUR PROCÉDÉ DE PRODUCTION

(30) Priorität: 01.03.2006 DE 102006009278
(43) Veröffentlichungstag der Anmeldung: 12.11.2008
(73) Patentinhaber: Justus-Liebig-Universität Gießen, 35390 Giessen (DE)
(72) Erfinder: SCHREINER, Peter, R., 35435 Wettenberg (DE); FOKIN, Andrey, A., 35398 Giessen (DE); WANKA, Lukas, 61206 Wöllstadt (DE); WOLFE, Derek, Athens, GA 30605 (US)
(74) Vertreter: Buchhold, Jürgen
(86) Internationale Anmeldenummer: PCT/EP2007/001431
(87) Internationale Veröffentlichungsnummer: WO 2007/101535

(56) Entgegenhaltungen:
- EP-A1- 0 392 059
- WO-A-2006/010362
- GERZON KOERT ET AL.: "The Adamantyl Group in Medicinal Agents. IV. Sedative Action of 3,5,7-Trimethyladamantane-1-carboxamide and Related Agents" JOURNAL OF MEDICINAL CHEMISTRY., Nr. 10, 1967, Seiten 603-606, XP002441196 USAMERICAN CHEMICAL SOCIETY. WASHINGTON.
- ELVIRA SHOKOVA ET AL.: "Adamantylation and Adamantylalkylation of Amides, Nitriles and Urea in Trifluoroacetic Acid" SYNTHESIS., 1997, Seiten 1034-1040, XP002441197 DEGEORG THIEME VERLAG, STUTTGART.
- YU. N. KLIMOCHKIN ET AL.: "Synthesis of Alkoxycarbonylaminoadamantanes and Acetylaminoadamantanes in Nitric Acid" BULLETIN OF THE ACADEMY OF SCIENCES OF THE USSR, Bd. 37, Nr. 4, 1988, Seiten 757-759, XP002441198 USSR
- DATABASE CAPLUS CHEMICAL ABSTRACTS SERVICE, COLUMBUS, OHIO, US; 19. Oktober 2005 (2005-10-19), XIAOJUN ZHANG: "Preparation of a Substituted symmetry adamantane Amine Derivatives" XP002441200 Database accession no. 2005:1117325 & CN 1 566 075 A (XIAOJUN ZHANG) 19. Januar 2005 (2005-01-19)

## Beschreibung

Gegenstand der Erfindung sind 1-Formamido-adamantanderivate und ein Verfahren zur direkten Formamidierung oder Acetamidierung von Adamantan oder von Adamantanderivaten. 1-Formamido-3,5-dimethyladamantan sowie Verfahren zu seiner Herstellung sind nicht Gegenstand der vorliegenden Erfindung.

1-Formamido- und 1-Acetamido-adamantanderivate sind wichtige Zwischenprodukte bei der Herstellung von Amino-adamantanen, bei denen ein tertiäres H-Atom des Adamantangrundgerüstes durch eine Aminogruppe substituiert ist. Amino-adamantane zeichnen sich durch interessante biologische Wirkungen aus. Das 1-Amino-3,5-dimethyladamantan hat bereits als Pharmazeutikum unter dem Wirkstoffnamen Memantine Anwendung gegen die Alzheimer Krankheit gefunden. Es besteht deshalb großes Interesse, für diese Verbindung und andere Amino-adamantane einfache Synthesemöglichkeiten zu schaffen, um die Wirksamkeiten weiterer Vertreter dieser Verbindungsklasse untersuchen zu können. Da nach den bisherigen Kenntnissen Amino-adamantane bevorzugt durch saure Hydrolyse von entsprechenden Amido-adamantanen zugänglich sind, stellte sich die Aufgabe, neue Amido-adamantane und Verfahren zu ihrer Herstellung zu entwickeln.

Bisher wurde zur Synthese von Amido-adamantanen ein Syntheseweg beschritten, bei dem halogenierte Adamantane als Ausgangssubstanzen verwendet werden. Das halogenierte Adamantan wurde dann mit einem Säureamid, z.B. Acetamid, unter Abspaltung von Halogenwasserstoff umgesetzt. Dieses Verfahren ist jedoch mit dem Nachteil verbunden, dass dabei halogenhaltige Nebenprodukte entstehen, deren Entsorgung umständlich ist und Kosten verursacht.

Es stellte sich deshalb die Aufgabe, ein Verfahren zu direkten Amidierung von Adamantanen aufzufinden. Unter einer "direkten" Amidierung wird also die Einführung einer Amidgruppe, z.B. einer Formamidgruppe oder Acetamidgruppe, in das Adamantanmolekül verstanden, ohne dass vorher noch eine Halogenierung stattfindet.

Die Darstellung von Acetamiden zyklischer und polyzyklischer Alkane an sich ist ein literaturbekanntes Verfahren. Meistens wird von bromierten Vorläufern ausgegangen, die dann in einer Ritter-Typ-Reaktion unter stark sauren Bedingungen mit einem Nitril oder Blausäure umgesetzt werden.

Zur Einführung von Formamid- bzw. Acetamidogruppen sind mehrere Verfahren bekannt. Haaf (Angew. Chem. 1961, 73, 144) stellte 1-Formamidoadamantan durch Umsetzen von Adamantan mit tert.-Butylkationen unter stark sauren Bedingungen unter Verwendung von Blausäure als Nucleophil her. In späteren Arbeiten von Haaf (Chem. Ber. 1963, 96(12), 3359 - 3369) wurde der Austausch von tertiären Carbonsäure- oder Estergruppen durch Amide mit Blausäure oder Nitrilen in konz. H₂SO₄ beschrieben. So wurde beispielsweise durch Umsetzung von Adamantan-1-carbonsäure mit 100%-iger Schwefelsäure und NaCN 1-Formamido-adamantan in 56% Ausbeute gewonnen, durch Umsetzen mit Acetonitril wurde 1-Acetamido-adamantan gewonnen. Gerzon et al. (J. Med. Chem 1963, 6, (6), 760 - 763) stellten Acetamide des Adamantans und einiger Derivate aus bromierten Vorläufern durch Umsetzung mit Nitrilen in konz. Schwefelsäure her. In Gerzon, K.; Tobias, D. J.; Holmes, R.E.; Rathbun, R.E.; Kattau, R.W., J. Med. Chem. 1967, 10, (4), 603 - 606 wurden Formamide des Adamantans aus den bromierten Vorläufern hergestellt. N-Acyl-1-Amino-3,5,7-Trimethyladamantane wurden hier durch Umsetzen von 1-Brom-3,5,7-Trimethyladamantan mit Amiden, beispielsweise Formamid, bei 125 - 130 °C hergestellt. In BE 648 581 196 40 703 wurden sowohl 1-Formamido-adamantan sowie 1-Acetamidoadamantan durch Zutropfen von konz. Schwefelsäure zu einer Mischung aus 1-Bromadamantan und dem entsprechenden Nitril oder Blausäure hergestellt. In NL 292 605 196 50 726 wurden daneben die Amino-adamantane mit Ag₂SO₄ und N-Methylamiden im Überschuss zu N-methylierten Adamantylamiden umgesetzt. In NL 6 604 975 196 61 024 wurden Aminoadamantane durch Amidierung von bromierten oder hydroxylierten Vorläufern mit Blausäure oder Nitrilen in 100 - 110%-iger Schwefelsäure hergestellt. Skoldinov et al. (Khimiko-Farmatsevticheskii Zhumal 1967, 1 (8), 22 - 26) stellten Diamino-diadamantyl-Verbindungen durch eine Ritter-Reaktion der bromierten Vorläufer in konz. Schwefelsäure her. Fonken et al. (J. Org. Chem. 1968, 33(8), 3201 - 3207) stellten 1-Formamidoadamantan aus dem Ameisensäure-Salz des 1-Aminoadamantans durch Umsetzung mit Acetanhydrid her. Aldrich et al. (J. Med. Chem. 1971, 14 (6), 535 - 543) stellten 1-Formamido-adamantan durch Umsetzung von 1-Bromadamantan mit Formamid in Gegenwart von Ag₂SO₄ dar. Jones und Mellor (Synthesis 1976, (1), 32 - 33) synthetisierten Formamide und Acetamide u. a. aus Adamantan und 1,3-Dimethyladamantan durch Bleitetraacetat-induzierte Ritter-Reaktion mit NaCN (Herstellung der Formamide) und Acetonitril (Herstellung der Acetamide). Druelinger et al. (J. Heterocycl. Chem. 1976, 13(5), 1001 - 1007) stellten 1-Formamido-adamantan durch photochemische Reaktion aus N-Adampntyloxaziridin her. In SU 408 546 197 61 205 wurde eine Variante der Herstellung von 1-Aminoadamantan aus 1-Formamido-adamantan beschrieben, das wiederum aus 1-Chloradamantan oder 1-Bromadamantan mit einem Überschuss von Formamid bei hohen Temperaturen hergestellt wurde. Olah et al. (Synthesis 1979, (4), 274 - 276) stellten-Acetamido-adamantane durch Umsetzung halogenierter Adamantane mit NOBF₄ in Acetonitril dar. Sasaki et al. (J. Org. Chem. 1981, 46(26), 5445 - 5447) stellten 1-Formamido-adamantan aus 1-Bromadamantan durch Umsetzung mit Trimethylsilylcyanid unter TiCl₄-Katalyse her. Die EP 89-106 657 198 90 414 beschreibt die Darstellung einer Serie verschieden substituierter 1-Acetamido- bzw. 1-Formamido-adamantan-Derivate aus den 1-Bromadamantanen als Vorläufer, so wurde beispielsweise hier 1-Brom-3-Phenyladamantan mit einem Überschuss von Formamid längere Zeit erhitzt, wobei sich in 80% Ausbeute das 1-N-Formamido-3-Phenyladamantan bildete.

Barton et al. (Tetrahedron 1993, 49(33), 7193 - 7214) stellten 1-Formamido-adamantan durch Erhitzen von 1-Aminoadamantan und Ethylformiat in DMSO her. Goel et al. (Tetrahedron Lett. 1996, 37(45), 8129 - 8132) beschreiben eine Erweiterung der Ritter-Reaktion zur Darstellung von Formamiden aus Alkoholen, u. a. Adamantan-1-ol, unter der Anwendung von Trimethylsilylcyanid. Kovalev et al. (Synthesis 1997, (9), 1034 - 1040) beschrieben die Darstellung von 1-Acylaminoadamantanen aus Adamantan-1-ol mit Nitrilen, Harnstoffen oder Carbonsäureamiden in heißer Trifluoressigsäure. In der russischen Patentanmeldung RU 98-116 071 199 80 824 wurden verschiedene 1-Amino-adamantanderivate, u. a. 1-Formamido-adamantan, durch Umsetzung von 1,3-Dehydroadamantan mit überschüssigem Amin (teilweise in Gegenwart von Bortrifluorid-Etherat als Katalysator) dargestellt.

Die weitaus meisten bekannten Methoden zur Amidierung verzweigter Kohlenwasserstoffe gehen von den Halogenalkanen oder den korrespondierenden Alkoholen aus. Zu deren Darstellung ist ein zusätzlicher Syntheseschritt, oft mit Aufreinigung der Zwischenprodukte, nötig. Bei der Bromierung der Kohlenwasserstoffe wird üblicherweise elementares Brom in großen Überschüssen verwendet, was die Umsetzungen teuer, gefährlich und hinsichtlich der Entsorgung der Bromabfälle unattraktiv macht. Die meisten literaturbekannten Verfahren zur Darstellung von Formamiden des Adamantans und seiner Derivate verwenden die sehr giftige Blausäure. Die literaturbekannten Verfahren der Amidierung von Alkoholen verwenden teils gefährliche (Trimethylsilylcyanid) und teure Reagenzien und Lösungsmittel (Trifluoressigsäure).

Die bekannten bromfreien Verfahren verwenden organische Cosolventien, die zu Verunreinigungen der Produkte führen bzw. das giftige, umweltgefährdende und reproduktionsgiftige Bleitetraacetat.

Die Darstellung von Stabilomeren wie Adamantan oder 1,3-Dimethyladamantan schließlich erfolgt kommerziell unter Anwendung großer Mengen metallhaltiger Lewis-Säuren wie beispielsweise wasserfreiem AlCl₃, was zu einem erheblichem Abfallaufkommen führt.

Ein Verfahren zur direkten und halogenfreien Amidierung von Adamantanderivaten, wie Adamantan-1-carbonsäuren oder 1,3-Dimethyladamantan unter der Verwendung preiswerter Reagenzien ist in der WO 2006/010362 beschrieben. Die direkte Darstellung von Formamiden des Adamantans, des 1,3-Dimethyladamantans oder anderer Adamantanderivate ohne vorherige Umsetzungen zu aktivierten Adamantanderivaten (beispielsweise zu den Alkoholen oder den Bromverbindungen) ist in der Literatur nicht beschrieben.

Eine in-situ-Darstellung des direkt zu amidierenden Kohlenwasserstoffs, wie beispielsweise Adamantan oder 1,3-Dimethyladamantan, in sauren Bedingungen mit anschließender Amidierung zum Formamid oder zu anderen Acylaminen ist in der Literatur nicht beschrieben.

Klimochkin et al. beschreiben in "Synthesis of Alkoxycarbonylaminoadamantanes and Acetylaminoadamantanes in Nitric Acid", Bulletin of the Academy of Sciences of the USSR, Bd. 37, Nr. 4, 1988, S. 757-759, XP002441198, USSR ein Verfahren zur Halogen-freien Herstellung von 1-Acetylaminoadamantanen durch Umsetzung von Adamantan mit Acetamid in Salpetersäure (d = 1.5). Hierbei wird eine Lösung von Adamantan in Salpetersäure bei einer Temperatur <30°C mit Acetamid versetzt. Anschließend wird das Reaktionsgemisch 2 h auf 120°C erhitzt.

Aufgabe der Erfindung ist es, vereinfachte Verfahren zur selektiven Einführung von Aminogruppen in Adamantan und dessen Derivate, zur Verfügung zu stellen. Dabei soll die vorherige Halogenierung der eingesetzten Ausgangsmaterialien vermieden werden. Ferner weist die hier beschriebene Methodik ein günstiges Verunreinigungsprofil auf, da sie in hohen Ausbeuten und mit geringem Anteil an Nebenprodukten verläuft und einige Verunreinigungen in den Ausgangsmaterialien toleriert. Eine in situ-Herstellung des Ausgangsmaterials aus preisgünstigen Vorläufern unter denselben Bedingungen ("Eintopf-Verfahren") soll ermöglicht werden. Die bromfreie Darstellung von für die nachfolgenden Syntheseoperationen besser geeigneten Formamiden soll ebenso ermöglicht werden.

Es wurde nun gefunden, dass diese Aufgabe durch ein Verfahren gelöst wird, bei dem zur direkten Formamidierung oder Acetamidierung Adamantan oder ein Adamantanderivat, 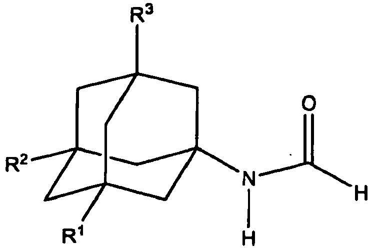 welches in 3, 5 und/oder 7-Stellung die Substituenten R¹, R² oder R³ trägt, die die folgenden Bedeutungen haben:
Wasserstoff, eine lineare oder verzweigte Alkyl-, Alkenyl- oder Alkinylgruppe mit bis zu 10 C-Atomen,
ein aliphatischer oder aromatischer, zyklischer oder heterozyklischer Kohlenwasserstoffrest mit bis zu 10 Kohlenstoffatomen,
oder ein Diamantoid, beispielsweise Diamantan, Triamantan, Tetramantane und Pentamantane
mit Formamid, Acetamid
oder Acetonitril in konzentrierten Säuren umgesetzt wird, wobei allerdings die direkte Formamidierung von 1,3-Dimethyladamantan ausgenommen ist
und wobei als konzentrierte Säuren 30 bis 70%-ige, insbesondere 65%-ige Salpetersäure und 90 bis 100%-ige, vor allem aber 95 bis 98%-ige Schwefelsäure verwendet wird. Es können aber auch 85 bis 100%-ige Phosphorsäue, Perchlorsäure, Dischwefelsäure oder Chlorschwefelsäure eingesetzt werden. Die Umsetzung erfolgt im Allgemeinen bei -40°C bis 50°C, bevorzugt bei 0°C. Bei dem erfindungsgemäßen Verfahren werden meistens Ausbeuten von 40 bis 95% erzielt.

Dieses Verfahren zeichnet sich nicht nur durch seine Halogenfreiheit aus, sondern weist auch ein günstigeres Verunreinigungsprofil auf, da es in hohen Ausbeuten und mit einem nur geringen Anteil an Nebenprodukten verläuft und dabei sogar Verunreinigungen in den Ausgangsmaterialien toleriert. Sogar eine in situ-Herstellung des Ausgangsmaterials aus preisgünstigen Vorläufern ist möglich, so dass in das gewünschte Endprodukt in einem "Eintopf"-Verfahren erhältlich ist. Dabei kann z.B. das umzusetzende Dialkyladamantan in situ aus einem perhydrierten Alkylcyclopentadien-Dimer durch kationische Umlagerung hergestellt und dann im gleichen Reaktionsgefäß die Amidierung des entstandenen Dialkyl-adamantans unter sauren Bedingungen durchgeführt werden.

Die Behandlung von perhydriertem Alkylcyclopentadien-Dimer mit konzentrierten Säuren ermöglicht die Darstellung des Ausgangsmaterials Dialkyladamantan in situ vor der Amidierung nach folgender Reaktion:

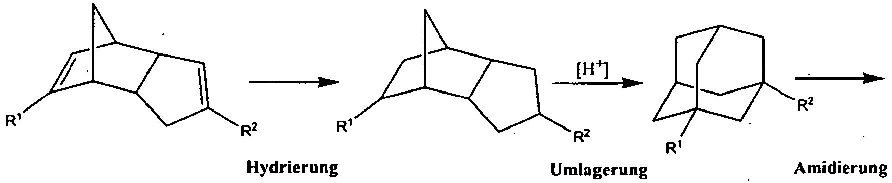

Das in der internationalen Anmeldung PCT/DE2005/001304 beschriebene Verfahren baut auf der Erzeugung von Carbokationen in konzentrierten Säuren auf, die durch ein Nukleophil, wie beispielsweise ein Nitril, abgefangen werden und bei wässriger Aufarbeitung in die korrespondierenden 1-Amido-adamantan-Derivate nach folgender Reaktion übergehen:

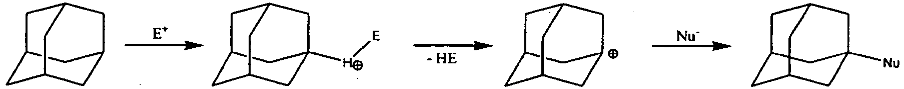

Die Optimierung der Reaktionsbedingungen bei der Synthese von 1-Formamido-adamantanen schafft die Möglichkeit, eine mildere Reaktionsführung unter völliger Vermeidung der Anwendung von Oleum oder 100%-iger Salpetersäure durchzuführen.

Die Erfindung wird durch das folgende Beispiel näher erläutert:

Direkte Amidierung von 1-Methyladamantan mit Formamid

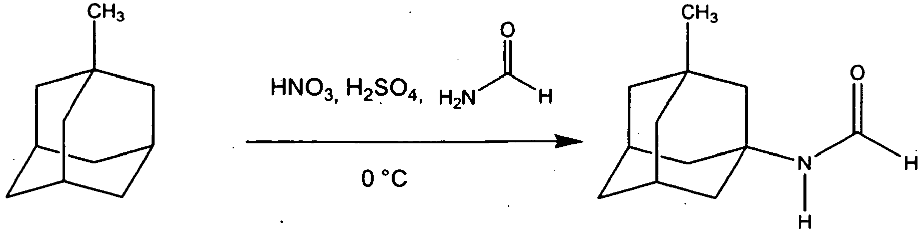

In einem ausgeheizten 100 mL-Rundkolben mit wirksamem Magnetrührer werden unter Kühlung auf 0 °C zu 1,5026 g ( 10 mmol) 1-Methyladamantan 1 mL technische Salpetersäure (65%) gegeben. Anschließend tropft man bei 0 °C innerhalb von 3 Stunden 25 mL technische Schwefelsäure (95 - 98%) zu. Man rührt 12 h bei 0 °C nach und gibt weitere 10 mL 95 - 98%ige Schwefelsäure hinzu. Nach weiteren 3 Stunden Rühren bei 0 °C wird die resultierende Lösung innerhalb einer Stunde unter Feuchtigkeitsausschluss und intensivem Rühren bei 0 °C zu 50 mL technischem Formamid getropft. Diese Mischung wird 30 min bei 0 °C und 120 min bei Raumtemperatur nachgerührt, wonach zuerst 100 mL Dichlormethan zugegeben werden. Man trennt die Phasen, extrahiert die wässrige Phase mit Dichlormethan (2 x 20 mL) und trocknet die vereinigten organischen Phasen mit Na₂SO₄. Nach dem Abfiltrieren des Trockenmittels wird die Lösung am Rotationsverdampfer weitgehend von Lösungsmitteln befreit. Das verbleibende, gelbliche Rohprodukt wird einer säulenchromatographischen Reinigung unterzogen (170 g SiO₂, Essigsäureethylester, Rf = 0.37). Nach sorgfältigem Abrotieren des Eluens erhält man 0,864 g (45%) 1-Formamido-3-methyladamantan als schwach gelbliches, viskoses Öl, das bei längerem Stehen in farblosen Nadeln durchkristallisiert.

**¹H NMR** (200 MHz, CDCl3): δ/ppm = 8.25 (d, J = 12.4 Hz) und 8.05 (d, J = 9.0 Hz), zusammen 1 H, NHCHO (2 Isomere); 6.62 (bs) und 5.33 (bs), zusammen 1 H, NHCHO (2 Isomere); 2.22-1.33 (m, 14 H, CH/CH₂ des Adamantans); 0.87 (s) und 0.85 (s), zusammen 3 H, -CH₃ (2 Isomere).

¹³C NMR (50 MHz, CDCl₃): δ/ppm = 162.6 und 160.4 (C=O), 53.0 und 51.6, 50.7 und 48.4, 43.3 und 43.2, 42.9 und 41.1, 35.5 und 35.1, 32.0 und 31.9, 30.4 und 30.3, 29.7 und 29.6 (Signale verdoppelt, da zwei Isomere).

IR (KBr): ṽ/cm⁻¹ = 3439, 3211, 3083, 2902, 2849, 1696, 1675, 1536, 1456, 1313.

MS: m/z = 193 (M⁺), 178, 148, 136, 122, 106, 92, 79

## Patentansprüche

1. Verfahren zur direkten Formamidierung oder Acetamidierung von Adamantan oder von einem Adamantanderivat der Formel II 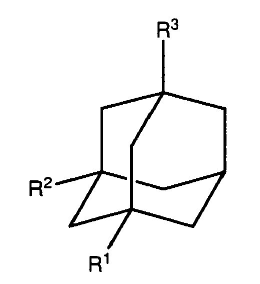 das in 3, 5 und/oder 7-Stellung die Substituenten R¹, R² oder R³ trägt, die die folgenden Bedeutungen haben:
Wasserstoff, eine lineare oder verzweigte Alkyl-, Alkenyl- oder Alkinylgruppe mit bis zu 6 C-Atomen,
ein aliphatischer oder aromatischer, zyklischer oder heterozyklischer Kohlenwasserstoffrest mit bis zu 10 Kohlenstoffatomen,
oder von einem Kohlenwasserstoff aus der Reihe der Diamantoide
**dadurch gekennzeichnet, dass** man das Adamantan, das Adamantanderivat oder das Diamantoid mit Formamid oder Acetamid in 30 bis 70%iger Salpetersäure und 90 bis 100%-iger Schwefelsäure, aber unter Vermeidung von SO₃-haltiger Schwefelsäure oder 100%-iger Salpetersäure umsetzt, wobei die direkte Formamidierung von 1,3-Dimethyladamantan ausgenommen ist

2. Verfahren nach Anspruch 1, **dadurch gekennzeichnet, dass** als Adamantanderivat ein Dialkyladamantan eingesetzt wird.

3. Verfahren nach den Ansprüchen 1 und 2, **dadurch gekennzeichnet, dass** man das Adamantan oder das Adamantanderivat vor der direkten Formamidierung oder Acetamidierung aus einem Cyclopentadien-Dimer oder einem seiner Derivate durch Perhydrierung in einer getrennten Reaktion oder in situ herstellt.

4. Verfahren nach den Ansprüchen 1 bis 3, **dadurch gekennzeichnet, dass** man das erhaltene Formamido- oder Acetamido-adamantan durch Hydrolyse in das entsprechende Amino-adamantan umwandelt.

## Claims

1. Method for the formamide or acetamide formation of adamantane or an adamantane derivative of the formula II 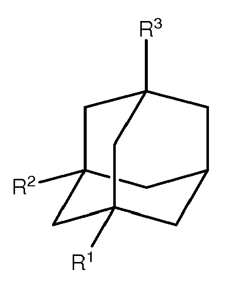 which carries the substituents R¹, R² or R³ in 3, 5 and/or 7 position, which are defined as follows:
hydrogen, a linear or branched alkyl, alkenyl or alkinyl group with up to 6 C atoms,
an aliphatic or aromatic, cyclic or heterocyclic hydrocarbon residue with up to 10 carbon atoms,
or from a hydrogen carbon from the diamondoid series,
wherein the adamantane, the adamantane derivative or the diamondoid is reacted with formamide or acetamide in 30% to 70% nitric acid and 90% to 100% sulphuric acid, but avoiding SO₃-containing sulphuric acids or 100% nitric acid, wherein the direct formamide formation of 1,3-dimethyladamantane is excluded.

2. Method according to claim 1, wherein a dialkyladamantane is used as an adamantane derivative.

3. Method according to claims 1 and 2, wherein the adamantane or the adamantane derivative is produced from a cyclopentadiene dimer or one of its derivatives via perhydrogenation in a separate reaction or in situ before the direct formamide or acetamide formation.

4. Method according to the claims 1 to 3, wherein the formamido-adamantane or acetamido-adamantane obtained is converted into the corresponding aminoadamantane by means of hydrolysis.

## Revendications

1. Procédé de formamidation directe ou d'acétamidation d'adamantane ou d'un dérivé d'adamantane de la formule II 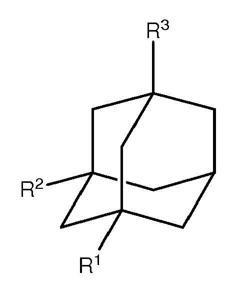 qui comporte les substituants R¹, R² ou R³ en position 3, 5 et/ou 7, qui ont les significations suivantes :
hydrogène, un groupe alkyle, alcényle ou alcynyle linéaire ou ramifié avec jusqu'à 6 atomes de carbone,
un résidu hydrocarboné aliphatique ou aromatique, cyclique ou hétérocyclique avec jusqu'à 10 atomes de carbone,
ou d'un hydrocarbure faisant partie de la série des diamantoïdes
**caractérisé en ce que** l'adamantane, le dérivé d'adamantane ou le diamantoïde fait réagir avec du formamide ou de l'acétamide dans 30 à 70% d'acide nitrique et 90 à 100% d'acide sulfurique, mais en évitant l'acide sulfurique contenant du SO₃ ou l'acide nitrique pur, à l'exception de la formamidation directe de 1,3-diméthyladamantane.

2. Procédé selon la revendication 1, **caractérisé en ce qu'**un dialkyleadamantane est utilisé comme dérivé d'adamantane.

3. Procédé selon les revendications 1 et 2, **caractérisé en ce que** l'on produit l'adamantane ou le dérivé d'adamantane avant la formamidation directe ou l'acétamidation à partir d'un dimère de cyclopentadiène ou un de ses dérivés par perhydrogénation dans une réaction distincte ou in situ.

4. Procédé selon les revendications 1 à 3, **caractérisé en ce qu'**on transforme le formamido- ou acetamidoadamantane obtenu par hydrolyse dans l'aminoadamantane correspondant.
